Europäisches Patentamt

European Patent Office

Office européen des brevets

(11) Publication number: 0 478 590 B1

# EUROPEAN PATENT SPECIFICATION

(45) Date of publication of patent specification: 27.07.94    (51) Int. Cl.5: **C07C 395/00, C07C 391/00**

(21) Application number: 90908608.4

(22) Date of filing: 08.06.90

(86) International application number:
**PCT/GB90/00894**

(87) International publication number:
**WO 90/15796 (27.12.90 90/29)**

(54) **METHOD FOR PREPARATION OF ORGANO-TELLURIUM AND SELENIUM COMPOUNDS.**

(30) Priority: **15.06.89 GB 8913799**

(43) Date of publication of application:
**08.04.92 Bulletin 92/15**

(45) Publication of the grant of the patent:
**27.07.94 Bulletin 94/30**

(84) Designated Contracting States:
**CH DE ES FR GB IT LI NL**

(56) References cited:
**EP-A- 0 285 834**
**GB-A- 2 185 256**
**GB-A- 2 213 148**
**GB-A- 2 213 149**

(73) Proprietor: **THE SECRETARY OF STATE FOR DEFENCE IN HER BRITANNIC MAJESTY'S GOVERNMENT OF THE UNITED KINGDOM OF GREAT BRITAIN AND NORTHERN IRELAND;**
**Whitehall**
**London SW1A 2HB(GB)**

(72) Inventor: **COLE-HAMILTON, David Chemistry Department**
**The Purdie Building**
**St Andrews**
**Fife KY16 95T(GB)**
Inventor: **McQUEEN, Alisdair, Ewan, David**
**37 Fife Park**
**St Andrews KY16 9UE(GB)**
Inventor: **MULLIN, John, Brian The Hoo Brockhill Road**
**West Malvern**
**Worcestershire WR14 4DL(GB)**

(74) Representative: **Beckham, Robert William**
**Defence Research Agency**
**Intellectual Property Department**
**DRA Farnborough**
**Farnborough, Hants. GU14 6TD (GB)**

EP 0 478 590 B1

**Description**

This invention relates to a method for the preparation of organo-tellurium and selenium compounds in which the tellurium or selenium is combined with two different organic groups. In particular the invention relates to a method for preparation of dialkyls or diaryls in which the tellurium or selenium is combined with two different alkyl or aryl groups, termed herein unsymmetrical dialkyls or diaryls.

The group 6 metals tellurium and selenium are important in semiconductor technology for example in the preparation of the infra-red detector material cadmium mercury telluride (CMT) and for use in light sensing switches respectively. Frequently these metals or in particular their compounds such as CMT are deposited on substrates by the process of Metal-Organic Vapour Phase Epitaxy (MOVPE) which involves vapour phase decomposition of a volatile organic compound of the metal, most usually a dialkyl. Dialkyls have the advantage that as well as being volatile they can be easily purified by formation of adducts which can be easily decomposed to form the pure dialkyl. For example GB 2 185 256 describes purification by formation of adducts with Group 15 compounds, and GB 2 213 149 describes purification by formation of adducts with Group 11 or group 12 metals compounds.

Unsymmetrical dialkyls of tellurium and selenium are believed to be advantageous for MOVPE because they have the potential for combining high volatility with low temperatures for deposition of the desired material. These properties have tended to be incompatible in dialkyls where the two alkyl groups are the same (ie "symmetrical dialkyls").

Although many methods of preparation of symmetrical dialkyls of tellurium and selenium are known (eg US 1578731; Synthetic Communications 12(3), 163-165, (1982); J Organometalic Chemistry 255, 61-70, (1983); GB 2 185 256; SU-A-371216; GB 2 213 148) little has been done to develop convenient methods of preparation of the unsymmetrical dialkyls.

Exchange reactions of the type:

$$R_2Te_2 \; + \; R'_2Te_2 \rightleftharpoons 2RTe_2R'$$
$$R_2Te \; + \; R'_2Te \rightleftharpoons 2R_2TeR'$$

are known, but neither of these is a useful synthetic pathway as the most volatile component of the mixture is a symmetrical component which will distil preferentially on attempting to separate the components by fractional distillation.

Organometallics 2, 305-307, (1983) describes a method for preparation of unsymmetrical dialkyl tellurides and phenyl-alkyl tellurides and selenides by the reaction of ditellurides diphenyldiselenide with a Grignard Reagent or an alkali metal alkyl in an ether solvent. A titration-type procedure is used, and clearly this introduces inconvenience. Furthermore Grignard Reagents and alkali metal alkyls are known to be quite sensitive to atmospheric hydrolysis, with the consequent risk of contamination. Grignard Reagents, such as ethyl magnesium bromide also introduce the possibility of contamination of the product by halogens, which can be very deleterious in semiconductor materials.

There is therefore a need for an improved method for preparation of unsymmetrical dialkyls and/or diaryls.

The method of this invention provides a method for the preparation of a tellurium or selenium compound of formula RaMRb characterised in that M is tellurium or selenium and Ra and Rb are different $C_1$-$C_{20}$ alkyl, alkenyl or aryl groups, wherein a compound of formula $(Ra)_2M_2$ is reacted with a compound of formula $(Rb)_2M$, in each compound M being the same, followed by isolation of the product RaMRb.

It is believed that (in the case of tellurium dialkyls at least) an intermediate ditelluride or diselenide RaMMRb is initially formed together with RaMRb, and this intermediate then decomposes on heating to form RaMRb and the metal M, ie:

(1)     $(Ra)_2M_2 \; + \; (Rb)_2M \rightarrow RaMRb \; + \; RaMMRb$

(2)     $RaMMRb \rightarrow RaMRb \; + \; M$

The overall reaction therefore being:

$(Ra)_2M_2 \; + \; (Rb)_2M \rightarrow 2RaMRb \; + \; M$

It is therefore preferred to carry out the method of the invention in two stages, firstly to allow the $(Ra)_2M_2$ and $(Rb)_2M$ to react at a lower temperature, especially at around room temperature (15-30°C), and then in a second stage to heat the reaction mixture to a higher temperature at which the intermediate decomposes.

In an unsymmetrical product Ra M Rb, clearly one of Ra or Rb may have a higher molecular weight than the other. It is known that generally the higher the molecular weight of the alkyl, alkenyl or aryl group in a tellurium or selenium alkyl, alkenyl or aryl, the lower is the volatility of the compound, and furthermore that ditellurides or diselenides are generally less volatile than the corresponding compound containing one tellurium or selenium atom.

Therefore in the method of the invention it is preferred that if possible the combinations of start-

ing materials $(Ra)_2M_2$ and $(Rb)_2M$ are chosen that the boiling point of the product Ra M Rb is lower than that of both starting material, so that the equilibrium of the reaction is encouraged toward Ra M Rb formation. It is also preferred that if Ra and Rb have different molecular weights, then Ra should have the lower, so that the effect of the lighter Ra in increasing volatility is to some extent compensated by its being present in the ditelluride or diselenide.

Preferably approximately stoichiometric quantities of the two reactants are used. It is also preferred that the first, lower temperature stage is performed in the dark. No solvent or diluent appears to be necessary for either step of the reaction, but in some cases (eg to encourage mixing of the reactants) conventional solvents such as ethers or alkanes may be used. It is preferred to carry out both stages of the reaction under an inert atmosphere such as nitrogen or in vacuo.

Advantageously after the first stage, solvents, volatile impurities and/or side products (if present) may be removed under reduced pressure, and then heat applied in the second, higher temperature, stage both to decompose the intermediate and to distil off the Ra M Rb product from the metal M.

The Ra M Rb product may then be further purified by conventional methods such as fractional distillation or via adduct formation as mentioned above. For example when Ra and Rb are both alkyl, traces of the symmetrical dialkyls may be formed, which can be easily removed by fractional distillation.

The method of the invention may of course be used as an improved method of preparation of an unsymmetrical ditelluride or diselenide of formula RaMMRb. To do this the above method of the invention is performed at a temperature at which decomposition of RaMMRb does not occur or occurs slowly enough for the RaMMRb product to be isolated, for example at about room temperature, followed by isolation of the product RaMMRb. The RaMMRb product may for example be isolated by removal of any RaMRb by fractional distillation. Although RaMMRb is likely to suffer from the disadvantage of being less volatile than RaMRb it may nevertheless find uses such as MOVPE in certain applications.

The method of the invention is applicable to the preparation of compounds in which Ra and Rb can be independently straight or branched alkyl or tertiary alkyl or alkenyl, or aryl hydrocarbon groups such as phenyl, alkyl- or alkenyl-, substituted phenyl, or phenyl- substituted alkyl or alkenyl groups. Preferred alkyl and alkenyl groups have short chains eg 5 or less carbon atoms, especially methyl, ethyl, n- or iso-propyl, n-, iso- or tert- butyl, and allyl.

Preferred RaRb combinations in RaMRb are those which result in highly volatile compounds which can easily be decomposed in MOVPE systems. Preferred combinations are Ra and Rb both alkyl, eg Ra being methyl and Rb being butyl, or one of Ra or Rb being alkyl and the other being alkenyl, eg Ra being methyl and Rb being allyl. The method of the invention appears to be suitable for the preparation of all RaRb combinations.

It appears that the unsymmetrical dialkyls produced by the method of this invention may combine low temperature deposition with volatility to allow tellurium containing semiconductors to be grown at relatively low temperatures with higher growth rates. Diallyltellluride has an estimated vapour pressure of only 3 mmHg (400 Pa) at 450°C and although it decomposes at a lower temperature than allylmethyltelluride the growth rate of CdTe between 240°C-260°C has been reported as 0.6 - 2.0 m/hr. Dimethyltelluride has a higher vapour pressure, 52 mmHg (6,9 kPa) at 25°C but temperatures of ca 500°C are required for growth of CdTe. By comparison, allylmethyltelluride combines a high vapour pressure of 25 mmHg (3,3 kPa) at 45°C with a good growth rate, 30 m/hr at 290°C.

Examples illustrating this invention will now be described.

Example 1

Preparation of methyltert-butyltellurium from dimethylditelluride and di-tert- butyltellurium

Dimethylditelluride (38.1 g) and di-tert-butyltellurium (29.9 g) were mixed in a flask and stirred under nitrogen at ambient temperature for one week. During this time the flask was kept in the dark. The volatile products of the reaction were removed by distillation under reduced pressure giving an orange oil. Distillation of this oil under an atmospheric of nitrogen gave methyltert-butyltellurium as a yellow liquid which boils at 119-122°C. Overall yield = 59%. The identity of the product was confirmed by $^1$H and $^{125}$Te NMR Spectroscopy. Traces of t-Bu$_2$ Te were removed by fractional distillation.

Example 2

Preparation of allylmethyltellurium from dimethylditelluride and diallyltellurium.

Diallyltellurium (15.98 g, 0.076 mol) and dimethylditelluride (25.04 g, 0.088 mol) were mixed in a flask under vacuum and left in the dark for 16 hours at room temperature. The volatile products were removed by warming the reaction mixture to 50°C and distilling at reduced pressure to give 25.63 g of an orange liquid. A second distillation at reduced pressure removed any excess dimethylditelluride giving 22.15 g (0.121 mol) of allylmethyltellurium as a yellow-orange liquid, an overall yield of 79.4 %. The identity of the product was confirmed by $^1$H and $^{125}$Te NMR spectroscopy.

Example 3

Preparation of methylisopropyltellurium from dimethyltelluride and di-isopropyltellurium.

Dimethyltelluride (5.1 g, 0.018 mol) and di-isopropyltellurium (3.8 g, 0.018 mol) were mixed and kept at room temperature for several days. Removal of the volatile fraction at reduced pressure with heating gave 4.9 g of a yellow oil. Analysis of this sample by $^{125}$Te and $^1$H nmr spectroscopy showed it to be MeTei-Pr contaminated with ca. 20% of i-Pr$_2$Te.

Example 4

Following the procedure of examples 1, 2 and 3 above isopropylisobutyl tellurium, methylethyltellurium, methylisopropyltellurium, and isopropylisobutyltellurium were also prepared.

**Claims**

1. A method for the preparation of a tellurium or selenium compound of formula RaMRb characterised in that M is tellurium or selenium and Ra and Rb are different $C_1$-$C_{20}$ alkyl, alkenyl or aryl groups, wherein a compound (Ra)$_2$M$_2$ is reacted with a compound of formula (Rb)$_2$M, in each compound M being the same, followed by isolation of the product RaMRb.

2. A method according to claim 1 wherein both Ra and Rb are alkyl groups.

3. A method according to claim 1 wherein one of Ra and Rb is alkyl and the other is alkenyl.

4. A method according to claim 1, 2 or 3 wherein Ra has a lower molecular weight than Rb.

5. A method according to claim 2, 3 or 4 wherein Ra and Rb contain 5 or less carbon atoms.

6. A method according to claim 5 wherein Ra is methyl and Rb is tert-butyl.

7. A method according to claim 5 wherein Ra is methyl and Rb is allyl.

8. A method according to claim 5 wherein Ra is methyl ad Rb is iso propyl.

9. A method according to claim 5 wherein Ra is isopropyl and Rb is isobutyl.

10. A method according to any one of claims 1 to 9 wherein the method is carried out in two steps, being a first lower temperature step followed by a second higher temperature step.

11. A method according to any one of claims 1 to 10 wherein M is tellurium.

12. A method for the preparation of a tellurium or selenium compound of formula RaMMRb Characterised in that M is tellurium or selenium and Ra and Rb are different $C_1$-$C_{20}$ alkyl, alkenyl or aryl groups, wherein a compound (Ra)$_2$M$_2$ is reacted with a compound (Rb)$_2$M, in each compound M being the same, followed by isolation of the product RaMMRb.

13. A method according to claim 12 wherein both Ra and Rb are alkyl groups.

14. A method according to claim 13 wherein Ra is methyl and Rb is butyl.

15. A method according to claim 13 wherein one of Ra and Rb is alkyl and the other is alkenyl.

16. A method according to claim 15 wherein Ra is methyl and Rb is allyl.

17. A method according to any one of claims 12 to 16 wherein M is tellurium.

**Patentansprüche**

1. Verfahren zur Herstellung einer Tellur- oder Selenverbindung der Formel RaMRb, **dadurch gekennzeichnet,** daß M Tellur oder Selen ist und Ra und Rb verschiedene $C_1$ - $C_{20}$ - Alkyl-, -Alkenyl- oder -Arylgruppen sind, wobei man eine (Ra)$_2$M$_2$ - Verbindung mit einer Verbindung der Formel (Rb)$_2$M mit gleichem M in jeder Verbindung reagieren läßt, worauf eine Abtrennung des Produkts RaMRb folgt.

2. Verfahren nach Anspruch 1, bei dem sowohl Ra als auch Rb Alkylgruppen sind.

3. Verfahren nach Anspruch 1, bei dem eines von Ra und Rb Alkyl ist und das andere Alkenyl ist.

4. Verfahren nach Anspruch 1, 2 oder 3, bei dem Ra ein niedrigeres Molekulargewicht als Rb hat.

5. Verfahren nach Anspruch 2, 3 oder 4, bei dem Ra und Rb 5 oder weniger Kohlenstoffatome enthalten.

6. Verfahren nach Anspruch 5, bei dem Ra Methyl ist und Rb Tert-butyl ist.

7. Verfahren nach Anspruch 5, bei dem Ra Methyl ist und Rb Allyl ist.

8. Verfahren nach Anspruch 5, bei dem Ra Methyl ist und Rb Isopropyl ist.

9. Verfahren nach Anspruch 5, bei dem Ra Isopropyl ist und Rb Isobutyl ist.

10. Verfahren nach irgendeinem der Ansprüche 1 bis 9, wobei das Verfahren in zwei Schritten, nämlich einem ersten Schritt bei niedrigerer Temperatur, auf den ein zweiter Schritt bei höherer Temperatur folgt, durchgeführt wird.

11. Verfahren nach irgendeinem der Ansprüche 1 bis 10, bei dem M Tellur ist.

12. Verfahren zur Herstellung einer Tellur- oder Selenverbindung der Formel RaMMRb,
**dadurch gekennzeichnet,**
daß M Tellur oder Selen ist und Ra und Rb verschiedene $C_1$ - $C_{20}$ - Alkyl-, -Alkenyl- oder Arylgruppen sind, wobei man eine $(Ra)_2 M_2$ - Verbindung mit einer $(Rb)_2 M$-Verbindung mit gleichem M in jeder Verbindung reagieren läßt, worauf eine Antrennung des Produkts RaMMRb folgt.

13. Verfahren nach Anspruch 12, bei dem sowohl Ra als auch Rb Alkylgruppen sind.

14. Verfahren nach Anspruch 13, bei dem Ra Methyl ist und Rb Butyl ist.

15. Verfahren nach Anspruch 13, bei dem eines von Ra und Rb Alkyl ist und das andere Alkenyl ist.

16. Verfahren nach Anspruch 15, bei dem Ra Methyl ist und Rb Allyl ist.

17. Verfahren nach irgendeinem der Ansprüche 12 bis 16, bei dem M Tellur ist.

**Revendications**

1. Procédé pour la préparation d'un composé de tellure ou de sélénium, de formule RaMRb, caractérisé en ce que M représente le tellure ou le sélénium et Ra et Rb représentent différents groupes alkyles, alcényles ou aryles en $C_1$ à $C_{20}$, procédé selon lequel on fait réagir $(Ra)_2 M_2$ avec un composé de formule $(Rb)_2 M$, le symbole M ayant le même sens dans chaque composé, et l'on isole ensuite le produit RaMRb.

2. Procédé selon la revendication 1, dans lequel les symboles Ra et Rb représentent tous deux des groupes alkyles.

3. Procédé selon la revendication 1, dans lequel l'un des symboles Ra et Rb représente un groupe alkyle et l'autre représente un groupe alcényle.

4. Procédé selon la revendication 1, 2 ou 3, dans lequel Ra a un poids moléculaire inférieur à celui de Rb.

5. Procédé selon la revendication 2, 3 ou 4, dans lequel Ra et Rb contiennent cinq ou moins de cinq atomes de carbone.

6. Procédé selon la revendication 5, dans lequel Ra représente un groupe méthyle et Rb un groupe tert.-butyle.

7. Procédé selon la revendication 5, dans lequel Ra représente un groupe méthyle et Rb représente un groupe allyle.

8. Procédé selon la revendication 5, dans lequel Ra représente un groupe méthyle et Rb représente un groupe isopropyle.

9. Procédé selon la revendication 5, dans lequel Ra représente un groupe isopropyle et Rb représente un groupe isobutyle.

10. Procédé selon l'une quelconque des revendications 1 à 9, dans lequel on met le procédé en oeuvre en deux étapes, la première étant une étape effectuée à basse température et suivie d'une seconde étape à température plus élevée.

11. Procédé selon l'une quelconque des revendications 1 à 10, dans lequel M représente le tellure.

**12.** Procédé pour la préparation d'un composé de tellure ou de sélénium de formule RaMRb, caractérisé en ce que M représente le tellure ou le sélénium et Ra et Rb représentent différents groupes alkyles, alcényles ou aryles en $C_1$ à $C_{20}$, dans lequel on fait réagir un composé $(Ra)_2M_2$ avec un composé $(Rb)_2M$, M ayant le même sens dans chaque composé, puis l'on isole le produit RaMRb.

**13.** Procédé selon la revendication 12, dans lequel les deux symboles Ra et Rb représentent chacun un groupe alkyle.

**14.** Procédé selon la revendication 13, dans lequel Ra représente un groupe méthyle et Rb représente un groupe butyle.

**15.** Procédé selon la revendication 13, dans lequel l'un des symboles Ra et Rb représente un groupe alkyle et l'autre représente un groupe alcényle.

**16.** Procédé selon la revendication 15, dans lequel Ra représente un groupe méthyle et Rb représente un groupe allyle.

**17.** Procédé selon l'une quelconque des revendications 12 à 16, dans lequel M représente le tellure.